# EUROPEAN PATENT APPLICATION

(11) **EP 4 411 358 A1**
(43) Date of publication of application: **07.08.2024**
(21) Application number: 22950024.4
(22) Date of filing: 30.09.2022
(51) Int. Cl.: G01N 27/00

(54) **COATING QUALITY INSPECTION METHOD, INSPECTION APPARATUS AND INSPECTION SYSTEM, AND STORAGE MEDIUM**

(30) Priority: 04.07.2022 CN 202210777935
(71) Applicant: Jiangsu Contemporary Amperex Technology Limited, Liyang City, Jiangsu 213300 (CN)
(72) Inventor: HE, Zhiyong, hangzhou, Jiangsu 213300 (CN); LUO, Changsheng, hangzhou, Jiangsu 213300 (CN); YANG, Weitao, hangzhou, Jiangsu 213300 (CN); ZHANG, Ming, hangzhou, Jiangsu 213300 (CN)
(74) Representative: Ran, Handong
(86) International application number: PCT/CN2022/123161
(87) International publication number: WO 2024/007463

(57) **Abstract**

A coating quality inspection method, relating to the technical field of coating. The inspection method comprises the following steps: detecting a coating area on a membrane to obtain millimeter wave data; acquiring edge zero lines (K0) at the two sides of the coating area, and according to the edge zero lines (KO) at the two sides, setting a main body area (S 1) of the coating area, wherein the main body area (S 1) comprises a central area (S2) located at the center of the main body area and edge areas (S3) located at the edges of the main body area; calculating a weight average value of the edge areas (S3) and a weight average value of the central area (S2); according to the weight average value of the edge areas (S3) and the weight average value of the central area (S2), calculating a first range value a between the weight average value of the edge areas and the weight average value of the central area, comparing the first range value a with a first set range value A, and according to a>A, determining that the surface coating of the membrane is abnormal; and/or calculating a weight range value b of the main body area (S 1), comparing the weight range value b with a second set range value B, and according to b>B, determining that the surface coating of the membrane is abnormal. According to the inspection method, the coating quality of the surface of an electrode sheet can be ensured, and thus the quality of a battery cell is ensured.

## Description

### CROSS-REFERENCE TO RELATED APPLICATIONS

This application claims priority to Chinese patent application No. 202210777935.5, filed on July 04, 2022, entitled "COATING QUALITY DETECTION METHOD, DETECTION APPARATUS, AND DETECTION SYSTEM, AND STORAGE MEDIUM", which is incorporated herein by reference in its entirety.

### TECHNICAL FIELD

This application relates to the field of coating technologies, and specifically discloses a coating quality detection method, detection apparatus, and detection system, and a storage medium.

### BACKGROUND

At present, as environmental problems become increasingly prominent, people begin to actively advocate a low-carbon economy. New energy vehicles have emerged due to worsening air quality. Manufacturers and consumers have also gradually recognized hybrid vehicles and pure electric vehicles, represented by new energy vehicles. As the main power source of new energy vehicles, traction batteries have become one of the core components of electric vehicles.

The coating process is a key process in the manufacture of traction battery cells, and coating quality is closely related to battery capacity, life, safety, and the like. At present, the coating process in the battery manufacturing field mainly relies on manual quality control and adjustment, with low control accuracy, slow adjustment speed, and low production efficiency, resulting in a large amount of material waste and increased labor costs, seriously affecting corporate profits. In addition, the defects related to the coating process are judged solely on the basis of experience and subjective perception of operators, which is also one of the reasons for the low production efficiency.

### SUMMARY

In view of the defects in the prior art, this application provides a coating quality detection method, so as to effectively solve the technical problems of poor coating quality detection effect and low detection efficiency.

According to a first aspect, this application provides a coating quality detection method, used for detecting quality of a coated membrane and including the following steps:
detecting a coating region on the membrane to obtain millimeter wave data;
obtaining edge zero lines on two sides of the coating region, and setting a primary zone of the coating region based on the edge zero lines on the two sides, where the primary zone includes a center zone located at the center thereof and edge zones located at the edges thereof;
calculating a weight average of the edge zones and a weight average of the center zone;
calculating a first extreme difference a between the weight average of the edge zones and the weight average of the center zone based on the weight average of the edge zones and the weight average of the center zone, comparing the first extreme difference a with a first specified extreme difference A, and determining, based on a>A, that coating on a surface of the membrane is abnormal; and/or
calculating a weight extreme difference b of the primary zone, comparing the weight extreme difference b with a second specified extreme difference B, and determining, based on b>B, that the coating on the surface of the membrane is abnormal.

In the technical solution of this application, by calculating the first extreme difference a between the weight average of the edge zones and the weight average of the center zone, and/or calculating the weight extreme difference b of the primary zone, and comparing the calculated results with the specified values, it can quickly, accurately, and effectively reflect the coating quality of a surface of the membrane, and determine whether the coating of the surface of the membrane is abnormal or not, so as to prevent the output of the membrane with an abnormal coating, thereby avoiding affecting the subsequent production process of the electrode plate, ensuring the coating quality of a surface of the electrode plate, and ensuring the quality of the battery cell.

In some embodiments of this application, the step of obtaining edge zero lines on two sides of the coating region on the membrane includes:
setting any point as an edge zero point of the coating region based on transition of a weight value of the point on the coating region to a specified weight value; and
determining an edge zero line based on a plurality of edge zero points on the same side of the coating region, where the plurality of edge zero points on the same side are jointly set on the edge zero line on the same side. During the coating, a coating weight value on two sides of the coating region of the membrane is transited from zero to a certain numerical value, which means that the surface of the membrane is coated with a material layer. A point at which the weight value starts to transition is an edge zero point, and a straight line in which a plurality of edge zero points are located is an edge zero line of the coating region, and a region formed between the edge zero lines on the two sides constitutes the coating region of the membrane. By determining the edge zero lines, an effective area of the coating region can be accurately defined, such that the coating quality of the surface of the membrane can be effectively detected.

In some embodiments of this application, the specified weight value is set to 250 mg. By setting the specified weight value to 250 mg, it can be ensured that the battery has sufficient capacity, and if a region has a weight value not reaching 250 mg, the surface is coated with no material layer, and the membrane cannot be used for cutting into electrode plates at a later stage, so the region of which the weight value does not reach 250 mg is excluded from the region under coating quality detection.

In some embodiments of this application, a line midway between the edge zero lines on the two sides is set as a center line, and a region formed by translating the center line towards the edge zero lines on the two sides by a length of L1 is the center zone, where 0<L1≤5 mm, which means that a maximum width size of the center zone in the coating region is 10 mm, so as to ensure the uniformity of the coating on the center zone after the setting.

In some embodiments of this application, regions formed by separately translating boundary lines on two sides of the primary zone towards the center of the membrane by a length of L2 are the edge zones, where 0<L2≤10 mm. By setting the regional extent of the edge zone, and by calculating the weight average of the edge zones and the first extreme difference a and comparing them with the specified values, it can effectively make a determination as to whether warping and/or thickening of the edge occurs in the coating region.

In some embodiments of this application, the edge zero lines on the two sides are separately translated towards the center of the membrane by a length of L3 to form thinning zones, where the thinning zones are arranged adjacent to the primary zone, and 0≤L3≤10 mm. By setting the regional extent of the thinning zone, the primary zone of the coating region can be limited, so as to determine whether a coating abnormality occurs in the primary zone by analyzing and calculating the primary zone.

In some embodiments of this application, a remaining region after the thinning zones between the edge zero lines on the two sides are excluded is set as the primary zone. The thinning zones are excluded from the coating region formed between the edge zero lines on the two sides, such that the remaining region forms the primary zone, and the coating quality of the surface of the membrane is determined by calculating the weight extreme difference b of the primary zone and performing comparison.

In some embodiments of this application, the first specified extreme difference A is set to 5.5 mg, and/or the second specified extreme difference B is set to 8 mg. By setting the first specified extreme difference A to 5.5 mg, and making the first extreme difference a between the weight average of the edge zones and the weight average of the center zone less than or equal to 5.5 mg, the safety of the battery cell can be effectively ensured. By making the weight extreme difference b of the primary zone less than or equal to 8 mg, scratches and/or dark traces in the coating region can be effectively prevented.

In some embodiments of this application, the determining, based on a>A, that coating on a surface of the membrane is abnormal includes determining that the coating on the surface of the membrane has warped edges and/or thick edges. By calculating the first extreme difference a and comparing it with the first specified extreme difference A, it can be effectively determined whether the coating on the surface of the membrane has warped edges and/or thick edges or not.

In some embodiments of this application, the determining, based on b>B, that coating on a surface of the membrane is abnormal includes determining that the coating on the surface of the membrane has dark traces and/or scratches. By calculating the weight extreme difference b of the primary zone and comparing it with the second specified extreme difference B, it can be effectively determined whether the coating on the surface of the membrane has dark traces and/or scratches or not.

In some embodiments of this application, the detection method further includes the following step:
performing difference operation on the millimeter wave data of the coating region. By performing difference operation on the millimeter wave data of the coating region, the overall weight of the coating region of the membrane can be effectively detected, thereby determining the capacity of the battery constituted after the membrane is formed into electrode plates.

In some embodiments of this application, after the determining that coating on a surface of the membrane is abnormal, the method further includes the following step:
issuing an alarm prompt, and/or marking a region on the surface of the membrane as abnormal coating. Upon comparison, it is determined that the coating on the surface of the membrane is abnormal, thereby issuing an alarm prompt to alert an operator of the defective coating region; and/or after it is determined that the coating on the surface of the membrane is abnormal, the membrane region with abnormal surface coating is marked, so as to remind the operator of the next process to pay attention to the region with abnormal coating, and prevent continuing to reprocess the membrane region with abnormal coating, avoiding wasting resources.

According to a second aspect, this application provides a coating quality detection apparatus configured to perform the coating quality detection method according to any one described above, where the detection apparatus includes:
a detection module, configured to detect a coating region on a membrane to obtain millimeter wave data;
an obtaining module, configured to: obtain edge zero lines on two sides of the coating region, and set a primary zone of the coating region based on the edge zero lines on the two sides, where the primary zone includes a center zone located at the center thereof and edge zones located at the edges thereof;
a calculation module, configured to: calculate a weight average of the edge zones and a weight average of the center zone, and calculate a first extreme difference a between the weight average of the edge zones and the weight average of the center zone based on the weight average of the edge zones and the weight average of the center zone, and configured to calculate a weight extreme difference b of the primary zone; and
a determining module, configured to: compare the first extreme difference a with a first specified extreme difference A, and determine, based on a>A, that coating on a surface of the membrane is abnormal, and/or configured to: compare the weight extreme difference b with a second specified extreme difference B, and determine, based on b>B, that the coating on the surface of the membrane is abnormal.

In some embodiments of this application, the detection module includes an X-ray surface density measuring instrument.

According to a third aspect, this application provides a coating quality detection system, where the detection system includes a processor and a memory storing computer program instructions; where:
when the computer program instructions are executed by the processor, the coating quality detection method according to any one described above is implemented.

According to a fourth aspect, this application provides a storage medium, where the storage medium stores computer program instructions, and when the computer program instructions are executed by a processor, the coating quality detection method according to any one described above is implemented.

The detection apparatus, the detection system, and the storage medium of this application are all capable of executing or implementing the coating quality detection method, and therefore they have the same technical effect as the coating quality detection method, and are not repeated herein.

The foregoing description is merely an overview of the technical solution of this application. For a better understanding of the technical means in this application such that they can be implemented according to the content of the specification, and to make the above and other objectives, features and advantages of this application more obvious and easier to understand, the following describes specific embodiments of this application.

### BRIEF DESCRIPTION OF DRAWINGS

Persons of ordinary skill in the art can clearly understand various other advantages and benefits by reading the detailed description of the preferred embodiments below. The accompanying drawings are merely intended to illustrate the preferred embodiments and are not intended to limit this application. Throughout the accompanying drawings, the same reference signs represent the same components. In the accompanying drawings:
FIG. 1 is a flowchart of a coating quality detection method according to an embodiment of this application;
FIG. 2 is a schematic diagram of a distribution structure of a coating region according to an embodiment of this application;
FIG. 3 is a data distribution diagram of millimeter wave data according to an embodiment of this application; and
FIG. 4 is a schematic diagram of a frame structure of a coating quality detection apparatus according to an embodiment of this application.

### DESCRIPTION OF EMBODIMENTS

The following describes in detail the embodiments of technical solutions of this application with reference to the accompanying drawings. The following embodiments are merely intended for a clearer description of the technical solutions of this application and therefore are used as just examples which do not constitute any limitations on the protection scope of this application.

It should be noted that, unless otherwise stated, the technical terms or scientific terms used in the embodiments of this application should be in the ordinary meaning as understood by persons skilled in the field to which the embodiments of this application belong.

In the description of the embodiments of this application, the orientations or positional relationships indicated by the technical terms "center", "longitudinal" "transverse", "length", "width", "thickness", "upper", "lower", "front", "rear", "left", "right", "perpendicular", "horizontal", "top", "bottom", "inside", "outside", "clockwise", "counterclockwise", "axial", "radial", "circumferential", and the like are based on the orientations or positional relationships as shown in the accompanying drawings. These terms are merely for ease and brevity of the description of the embodiments of this application rather than indicating or implying that the apparatuses or components mentioned must have specific orientations, or must be constructed or manipulated according to specific orientations, and therefore shall not be construed as any limitations on embodiments of this application.

In addition, the technical terms "first", "second", and the like are merely for the purpose of description, and shall not be understood as any indication or implication of relative importance or any implicit indication of the number of technical features indicated. In the description of this application, "a plurality of" means at least two unless otherwise specifically stated.

In the description of the embodiments of this application, unless otherwise specified and defined explicitly, the terms "mounting", "connection", "join", and "fastening" should be understood in their general senses. For example, they may refer to a fixed connection, a detachable connection, or an integral connection, may refer to a mechanical connection or an electrical connection, and may refer to a direct connection, an indirect connection via an intermediate medium, an internal communication between two elements, or an interaction between two elements. Persons of ordinary skill in the art can understand specific meanings of these terms in this application as appropriate to specific situations.

In the description of the embodiments of this application, unless otherwise expressly specified and defined, the first feature being "above" or "below" the second feature may mean that the first feature is in direct contact with the second feature or may mean that the first feature and the second feature come into contact indirectly through an intermediary. Moreover, that the first feature is "above", "over", or "on" the second feature may mean that the first feature is directly above or obliquely above the second feature or simply mean that the first feature has a higher level than the second feature. That the first feature is "below", "beneath", and "under" the second feature may mean that the first feature is directly below or obliquely below the second feature or simply mean that the first feature has a lower level than the second feature.

Currently, from a perspective of the market development, application of traction batteries is being more extensive. Traction batteries have been not only used in energy storage power supply systems such as hydroelectric power plants, thermal power plants, wind power plants, and solar power plants, but also widely used in many other fields including electric transportation tools such as electric bicycles, electric motorcycles, and electric vehicles, military equipment, and aerospace.

The inventors have observed that during the production of the battery, the coating quality of the electrode plate is closely related to battery capacity, life, safety, and the like. At present, the coating process in the battery manufacturing field mainly relies on manual quality control and adjustment, with low control accuracy, slow adjustment speed, and low production efficiency, resulting in a large amount of material waste and increased labor costs, seriously affecting corporate profits. In addition, the defects related to the coating process are judged solely on the basis of experience and subjective perception of operators, which is also one of the reasons for the low production efficiency.

To solve the technical problems of poor coating quality detection effect and low detection efficiency, the applicant has found through research that by dividing the coating region into a plurality of zones, calculating weight extreme differences of the divided zones, or calculating weight extreme difference between different zones, and comparing the calculated results with specified values, it can quickly, accurately, and effectively reflect the coating quality of the surface of the membrane, so as to prevent the output of the membrane with an abnormal coating, thereby avoiding affecting the subsequent production process of the electrode plate, ensuring the coating quality of a surface of the electrode plate, and ensuring the quality of the battery cell.

On the basis of the foregoing considerations, the inventors, after in-depth research, have designed a method for controlling coating quality. By dividing the coating region into thinning zones and a primary zone, where the primary zone includes a center zone located in the center thereof and edge zones located at edges thereof, and calculating a first extreme difference a between a weight average of the edge zones and a weight average of the center zone, and/or calculating a weight extreme difference b of the primary zone, and comparing calculated results with specified values, it can quickly, accurately, and effectively reflect the coating quality of a surface of the membrane, and determine whether the coating of the surface of the membrane is abnormal or not, so as to prevent the output of the membrane with an abnormal coating, thereby avoiding affecting the subsequent production process of the electrode plate, ensuring the coating quality of a surface of the electrode plate, and ensuring the quality of the battery cell.

This application provides a coating quality detection method, detection apparatus, and detection system, and a storage medium, and the detection method can be used for coating a membrane of anode electrode plates, and can also be used for coating a membrane of cathode electrode plates. The coated membrane can be used for any battery, for example, battery modules and battery packs, or for primary and secondary batteries, for example, secondary batteries including lithium-ion batteries, sodium-ion batteries, lithium sulfur batteries, magnesium-ion batteries, and the like. Such batteries are applicable to various electric devices that use batteries, for example, mobile phones, portable devices, notebook computers, electric bicycles, electric toys, electric tools, electric vehicles, ships, and spacecrafts. For example, spacecrafts include airplanes, rockets, space shuttles, and spaceships. The batteries are used to provide electrical energy for the above-mentioned electrical devices.

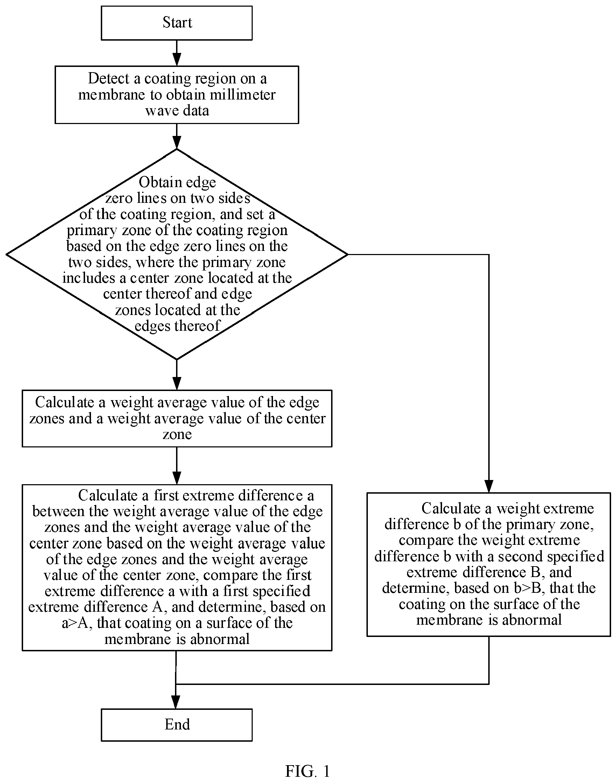

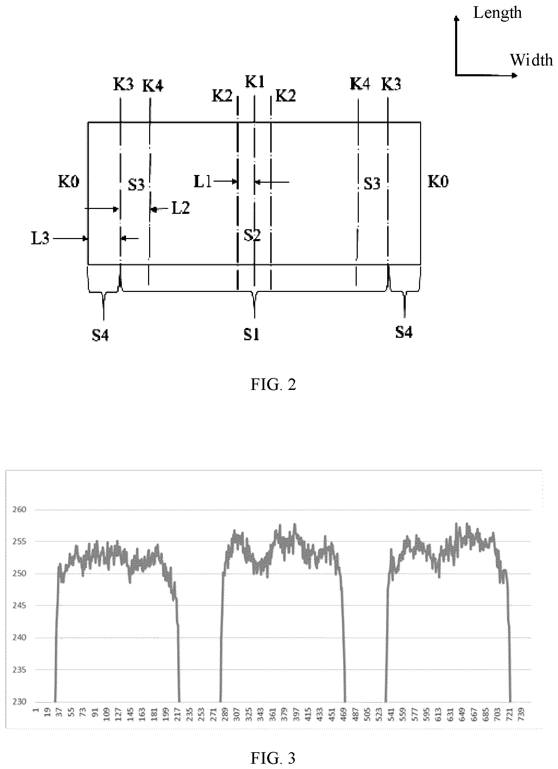

Referring to FIG. 1, FIG. 2, and FIG. 3 together, FIG. 1 is a flowchart of a coating quality detection method according to an embodiment of this application; FIG. 2 is a schematic diagram of a distribution structure of a coating region according to an embodiment of this application; and FIG. 3 is a data distribution diagram of millimeter wave data according to an embodiment of this application. According to a first aspect, this application provides a coating quality detection method, used for detecting quality of a coated membrane and including the following steps:
detecting a coating region on the membrane to obtain millimeter wave data;
obtaining edge zero lines K0 on two sides of the coating region, and setting a primary zone S1 of the coating region based on the edge zero lines K0 on the two sides, where the primary zone S1 includes a center zone S2 located at the center thereof and edge zones S3 located at the edges thereof;
calculating a weight average of the edge zones S3 and a weight average of the center zone S2;
calculating a first extreme difference a between the weight average of the edge zones S3 and the weight average of the center zone S2 based on the weight average of the edge zones S3 and the weight average of the center zone S2, comparing the first extreme difference a with a first specified extreme difference A, and determining, based on a>A, that coating on a surface of the membrane is abnormal; and/or
calculating a weight extreme difference b of the primary zone S1, comparing the weight extreme difference b with a second specified extreme difference B, and determining, based on b>B, that the coating on the surface of the membrane is abnormal.

Specifically, electrode plates are cut from a coated membrane, where a width size of the membrane is greater than a width size of an electrode plate formed, and the width size of the same membrane is a sum of width sizes of several electrode plates formed, and several electrode plates can be formed after the membrane is cut along a width direction of the membrane. Therefore, when coating is performed on the same membrane, several coating lines are provided at intervals along the width direction of the membrane, and the membrane is cut into several electrode plates after the coating. As the coating is carried out along a length direction of the membrane and the coating direction is perpendicular to the width direction, obvious weight transitions occur at the edges on two sides of the coating region in its width direction, the region before occurrence of the weight transitions cannot be used for making finished electrode plates, and it is necessary to remove this portion of the region without having to analyze the quality of the surface thereof. Therefore, it is necessary to obtain the edge zero lines K0 on the two sides of the coating region and set the primary zone S1 of the coating region based on the edge zero lines K0 on the two sides. The primary zone S1 includes a center zone S2 located at the center thereof and edge zones S3 located at the edges thereof, and obtain millimeter wave data corresponding to the primary zone S1, the center zone S2, and the edge zones S3 respectively.

After the division of the coating region is completed, the coating quality of the surface of the membrane can be determined in two ways. One way is to calculate the weight average of the edge zones S3 and the weight average of the center zone S2, calculate a first extreme difference a based on the weight average of the edge zones S3 and the weight average of the center zone S2, compare the first extreme difference a with a first specified extreme difference A, and determine, based on a>A, that coating of a surface of the membrane is abnormal. The other way is to calculate a weight extreme difference b of the primary zone S1, compare the weight extreme difference b with a second specified extreme difference B, and determine, based on b>B, that the coating on the surface of the membrane is abnormal. For the foregoing two determining ways, only if one of a>A or b>B is satisfied, it can be determined that the coating on the surface the membrane is abnormal.

Specifically, the weight average of the edge zones S3 and the weight average of the center zone S2 can be calculated based on the millimeter wave data in FIG. 3, and the first extreme difference a between the weight average of the edge zone S3 and the weight average of the center zone S2 and the weight extreme difference b the primary zone S1 can be calculated based on the weight average of the edge zone S3 and the weight average of the center zone S2. The specific difference operation method is a conventional technical means in the field and is not described herein.

Based on the technical solution of this application, by calculating the first extreme difference a between the weight average of the edge zones S3 and the weight average of the center zone S2, and/or calculating the weight extreme difference b of the primary zone S1, and comparing the calculated results with the specified values, it can quickly, accurately, and effectively reflect the coating quality of a surface of the membrane, so as to prevent the output of the membrane with an abnormal coating, thereby avoiding affecting the subsequent production process of the electrode plate, ensuring the coating quality of a surface of the electrode plate, and ensuring the quality of the battery cell.

In some embodiments of this application, the step of obtaining edge zero lines on two sides of the coating region on the membrane includes:
setting any point as an edge zero point of the coating region based on transition of a weight value of the point on the coating region to a specified weight value; and
determining an edge zero line K0 based on a plurality of edge zero points on the same side of the coating region, where the plurality of edge zero points on the same side are jointly set on the edge zero line K0 on the same side.

Specifically, the edge zero line K0 may be obtained based on a point at which the weight value transits. When a weight value of any point on the coating region transits to a specified weight value, the point can be determined as the edge zero point of the coating region. A line at which the edge zero point is located is the edge zero line K0. As the coating direction runs in a straight line along a length direction of the membrane, the edge zero points on the same side of the coating region are roughly on the same straight line, to be specific, the edge zero points on the same side are jointly arranged on the edge zero line K0 on the same side, and the edge zero line K0 is parallel to the coating direction, that is, the length direction of the membrane.

During the coating, a coating weight value on two sides of the coating region of the membrane is transited from zero to a certain numerical value, which means that the surface of the membrane is coated with a material layer. A point at which the weight value starts to transition is an edge zero point, and a straight line in which a plurality of edge zero points are located is an edge zero line K0 of the coating region, and a region formed between the edge zero lines K0 on the two sides constitutes the coating region of the membrane. By determining the edge zero lines K0, an effective area of the coating region can be accurately defined, such that the coating quality of the surface of the membrane can be effectively detected. Therefore, when the coating quality of the surface of the membrane is being detected, the coating region of the membrane needs to be determined first.

Referring to FIG. 2 and FIG. 3 together, in some embodiments of this application, the specified weight value is set to 250 mg.

Specifically, in some embodiments of this application, as shown in FIG. 3, three rows of coating regions are spaced on the same membrane in the width direction. In FIG. 3, the horizontal coordinate is the width size of the membrane in mm, and the vertical coordinate is the weight value in mg. It can be clearly seen from FIG. 3 that transition occurs when the weight values of edges of the same coating region at the two sides reach 250 mg, and thus 250 mg is defined as the specified weight value for weight value transition. Points at which the weight values of the edges at the two sides reach 250 mg are the edge zero points, and straight lines at which the edge zero points are located are the edge zero lines K0.

By setting the specified weight value to 250 mg, it can be ensured that the battery formed has sufficient capacity, and if a region has a weight value not reaching 250 mg, the surface is coated with no material layer, and the membrane cannot be used for cutting into electrode plates at a later stage, so the region of which the weight value does not reach 250 mg is excluded from the region under coating quality detection.

As shown in FIG. 2, in some embodiments of this application, a line midway between the edge zero lines K0 on the two sides is set as a center line K1, and a region formed by translating the center line K1 towards the edge zero lines K0 on the two sides by a length of L1 is the center zone S2, where 0<L1≤5 mm.

Specifically, the center line K1 is in the symmetric line of the edge zero lines K0 on the two sides, that is, the center position of the coating region. The center line K1 is translated by 0-5 mm towards the edge zero lines K0 on the two sides to first boundary lines K2, and a region formed between the two first boundary lines K2 is the center zone S2 of the coating region,
which means that a maximum width size of the center zone S2 in the coating region is 10 mm, so as to ensure the uniformity of the coating on the center zone S2 after the setting. Specifically, the center line K1 may be translated by 5 mm towards the edge zero lines K0 on the two sides, so as to ensure the uniformity of the coating of the center zone S2 to the greatest extent.

As shown in FIG. 2, in some embodiments of this application, regions formed by separately translating boundary lines on two sides of the primary zone S1 towards the center of the membrane by a length of L2 are the edge zones S3, where 0<L2≤10 mm.

Specifically, second boundary lines K3 are moved towards the center of the membrane to third boundary lines K4, and regions formed between the second boundary lines K3 and the third boundary lines K4 are the edge zones S3. A distance between the second boundary line K3 and the third boundary line K4 is greater than or equal to 0 and less than or equal to 10 mm.

By setting the regional extent of the edge zone S3, and by calculating the weight average of the edge zones S3 and the first extreme difference a and comparing them with the specified values, it can effectively make a determination as to whether warping and/or thickening of the edge occurs in the coating region.

As shown in FIG. 2, in some embodiments of this application, the edge zero lines K0 on the two sides are separately translated towards the center of the membrane by a length of L3 to form thinning zones S4, where the thinning zones S4 are arranged adjacent to the primary zone S1, and 0≤L3≤10 mm.

Specifically, the edge zero lines K0 is moved toward the center of the membrane to the second boundary lines K3, and regions formed between the second boundary lines K3 and the edge zero lines K0 are the thinning zones S4. A distance between the second boundary line K3 and the edge zero lines K0 is greater than or equal to 0 and less than or equal to 10 mm.

By setting the regional extent of the thinning zone S4, the primary zone S1 of the coating region can be limited, so as to determine whether a coating abnormality occurs in the primary zone S1 by analyzing and calculating the primary zone S1.

As shown in FIG. 2, in some embodiments of this application, a remaining region after the thinning zones S4 between the edge zero lines K0 on the two sides are excluded is set as the primary zone S1.

Specifically, a region between the edge zero lines K0 on the two sides is the coating region of the membrane, a region between the edge zero line K0 and second boundary line K3 on any side is the thinning zone S4, and a remaining region after the thinning zones S4 are excluded from the coating region is the primary zone S1, to be specific, the region between the second boundary lines K3 on the two sides is the primary zone S1.

The thinning zones S4 are excluded from the coating region formed between the edge zero lines K0 on the two sides, such that the remaining region forms the primary zone S1, and the coating quality of the surface of the membrane is determined by calculating the weight extreme difference b of the primary zone S1 and performing comparison.

Still as shown in FIG. 2, in some embodiments of this application, the first specified extreme difference A is set to 5.5 mg, and/or the second specified extreme difference B is set to 8 mg.

By setting the first specified extreme difference A to 5.5 mg, and making the first extreme difference a between the weight average of the edge zones S3 and the weight average of the center zone S2 less than or equal to 5.5 mg, the warped edges and/or thick edges in the coating region can be effectively prevented. By making the weight extreme difference b of the primary zone S1 less than or equal to 8 mg, scratches and/or dark traces in the coating region can be effectively prevented.

Still as shown in FIG. 2, in some embodiments of this application, the determining, based on a>A, that coating on a surface of the membrane is abnormal includes determining that the coating on the surface of the membrane has warped edges and/or thick edges.

Specifically, the edge zones S3 are located at the edges of the primary zone S1 on the two sides and are prone to the warped edges and/or thick edges after the membrane is cut into electrode plates. Therefore, it is necessary to calculate the weight average of the edge zones S3 and the weight average of the center zone S2, and compare a first extreme difference a between the two with the first specified extreme difference A, so as to effectively determine whether the coating on the surface of the membrane has warped edges and/or thick edges or not. The weight average of the edge zones S3 and the weight average of the center zone S2 can be determined from the millimeter wave data in FIG. 3, and the calculation of the weight extreme difference based on the weight average is a conventional technical means in the art, so the calculation of the first extreme difference a is not described in detail.

Still as shown in FIG. 2, in some embodiments of this application, the determining, based on b>B, that coating on a surface of the membrane is abnormal includes determining that the coating on the surface of the membrane has dark traces and/or scratches.

Specifically, although the primary zone S1 is located in the middle of the coating region of the membrane and is not susceptible to the warped edges and/or thick edges, dark traces and/or scratches may present on the surface of the membrane due to the presence of particles in the coating process. Therefore, it is necessary to calculate the weight extreme difference b of the primary zone S1 and compare it with the second specified extreme difference B, so as to effectively determine whether the coating on the surface of the membrane has dark traces and/or scratches or not. The millimeter wave data in FIG. 3 can be used to determine weight values at various positions in the primary zone S1, and the calculation of the weight extreme difference based on the corresponding weight values is a conventional technical means in the field, so the calculation of the weight extreme difference b is not described in detail.

In some embodiments of this application, the detection method further includes the following step:
performing difference operation on the millimeter wave data of the coating region.

Specifically, the performing difference operation on the millimeter wave data of the coating region includes performing difference operation on an overall region of the coating region, to be specific, performing difference operation on the overall region including the thinning zones S4 and the primary zone S1.

By performing difference operation on the millimeter wave data of the coating region, the overall weight of the coating region of the membrane can be effectively detected, thereby determining the capacity of the battery constituted after the membrane is formed into electrode plates.

In some embodiments of this application, after the determining that coating on a surface of the membrane is abnormal, the method further includes the following step:
issuing an alarm prompt, and/or marking a region on the surface of the membrane as abnormal coating.

Specifically, after it is determined that the coating on the surface of the membrane is abnormal, an alarm may be issued in various forms, for example, sound, light, or vibration, so as to alert an operator of an abnormal point in the coating region; and/or after it is determined that the coating on the surface of the membrane is abnormal, an abnormally coated region may be marked by a marking device so as to alert the operator of the next process to pay attention to the abnormally coated region, and the abnormally coated membrane may be handled accordingly so as to prevent the defective membrane from outputting into the next process, avoiding wasting resources.

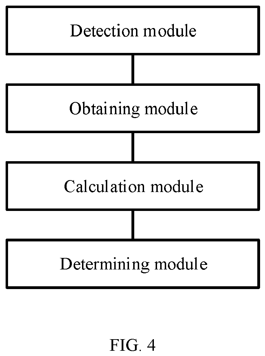

According to a second aspect, this application provides a coating quality detection apparatus configured to perform the coating quality detection method according to any one of the foregoing embodiments. Referring to FIG. 2, FIG. 3, and FIG. 4 together, FIG. 4 is a schematic diagram of a frame structure of a coating quality detection apparatus according to an embodiment of this application. In some embodiments of this application, the detection apparatus includes:
a detection module, configured to detect a coating region on a membrane to obtain millimeter wave data;
an obtaining module, configured to: obtain edge zero lines K0 on two sides of the coating region, and set a primary zone S 1 of the coating region based on the edge zero lines K0 on the two sides, where the primary zone S 1 includes a center zone S2 located at the center thereof and edge zones S3 located at the edges thereof;
a calculation module, configured to: calculate a weight average of the edge zones S3 and a weight average of the center zone S2, and calculate a first extreme difference a between the weight average of the edge zones S3 and the weight average of the center zone S2 based on the weight average of the edge zones S3 and the weight average of the center zone S2, and configured to calculate a weight extreme difference b of the primary zone S 1; and
a determining module, configured to: compare the first extreme difference a with a first specified extreme difference A, and determine, based on a>A, that coating on a surface of the membrane is abnormal, and/or configured to: compare the weight extreme difference b with a second specified extreme difference B, and determine, based on b>B, that the coating on the surface of the membrane is abnormal.

Specifically, the detection module sends detected millimeter wave data to the obtaining module. The obtaining module obtains edge zero lines K0 on the two sides of the coating region based on the millimeter wave data, sets a primary zone S 1 of the coating region based on the edge zero lines K0 on the two sides, where the primary zone S 1 includes a center zone S2 located in the center thereof and edge zones S3 located at the edges thereof, and sends the foregoing information to the calculation module. The calculation module calculates a weight average of the edge zones S3 and a weight average of the center zone S2 based on the millimeter wave data corresponding to each of the edge zones S3 and the center zone S2, calculates a first extreme difference a between the weight average of the edge zones S3 and the weight average of the center zone S2 based on the weight average of the edge zones S3 and the weight average of the center zone S2, and/or calculates a weight extreme difference b based on the millimeter wave data corresponding to the primary zone S1, and sends the calculated first extreme difference a and/or the weight extreme difference b to the determining module. The determining module compares the first extreme difference a with a first specified extreme difference A, and determines, based on a>A, that coating on a surface of the membrane is abnormal, and/or compares the weight extreme difference b with a second specified extreme difference B, and determines, based on b>B, that the coating on the surface of the membrane is abnormal.

Based on the technical solution of this application, by calculating the first extreme difference a between the weight average of the edge zones S3 and the weight average of the center zone S2, and/or calculating the weight extreme difference b of the primary zone S 1, and comparing the calculated results with the specified values, it can quickly, accurately, and effectively reflect the coating quality of a surface of the membrane, and determine whether the coating of the surface of the membrane is abnormal or not, so as to prevent the output of the membrane with an abnormal coating, thereby avoiding affecting the subsequent production process of the electrode plate, ensuring the coating quality of a surface of the electrode plate, and ensuring the quality of the battery cell.

In some embodiments of this application, the detection module includes an X-ray surface density measuring instrument.

Specifically, millimeter wave data of the coating region of the membrane may be detected by the X-ray surface density measuring instrument. The X-ray surface density measuring instrument utilizes the absorption and backscattering effects of X-rays as they penetrate a material to achieve non-contact measurement of a surface density of a film-like material, that is, millimeter wave data. When X-rays penetrate a material, they are reflected, scattered, and absorbed by the material, resulting in a certain attenuation of the intensity of the penetrating rays relative to the intensity of the incident rays. An attenuation ratio has a negative exponential relationship with the thickness/density of an object being penetrated. By measuring the intensities of the rays before and after penetration, the surface density of the substance can be inferred.

According to a third aspect, this application provides a coating quality detection system, where the detection system includes a processor and a memory storing computer program instructions; where
when the computer program instructions are executed by the processor, the coating quality detection method according to any one of the foregoing embodiments is implemented.

According to a fourth aspect, this application provides a storage medium, where the storage medium stores computer program instructions, and when the computer program instructions are executed by a processor, the coating quality detection method according to any one of the foregoing embodiments is implemented.

The detection system and the storage medium of this application are all capable of implementing the coating quality detection method, and therefore they have the same technical effect as the coating quality detection method, and are not repeated herein.

An embodiment of this application provides a coating quality detection method, used for detecting quality of a coated membrane and including the following steps:
detecting millimeter wave data of a coating region on a membrane, and performing difference operation on the millimeter wave data of the coating region;
obtaining edge zero lines K0 on two sides of the coating region, and setting a primary zone S 1 of the coating region based on the edge zero lines K0 on the two sides, where the primary zone S1 includes a center zone S2 located at the center thereof and edge zones S3 located at the edges thereof; transiting a specified weight value at any point on the coating region to a specified weight value, where the specified weight value is set to 250 mg, and the point is set as an edge zero point of the coating region; and determining an edge zero line K0 based on a plurality of edge zero points on the same side of the coating region, where the plurality of edge zero points on the same side are jointly set on the edge zero line K0 on the same side; where a line midway between the edge zero lines K0 on the two sides is set as a center line K1, and a region formed by translating the center line K1 towards the edge zero lines K0 on the two sides by a length of L1 is the center zone S2, where 0<L1≤5 mm, regions formed by separately translating boundary lines on two sides of the primary zone S 1 towards the center of the membrane by a length of L2 are the edge zones S3, where 0<L2≤10 mm; the edge zero lines K0 on the two sides are separately translated towards the center of the membrane by a length of L3 to form thinning zones S4, where the thinning zones S4 are arranged adjacent to the primary zone S1, and 0≤L3≤10 mm; and a remaining region after the thinning zones S4 between the edge zero lines K0 on the two sides are excluded is set as the primary zone S1;
calculating a weight average of the edge zones S3 and a weight average of the center zone S2;
calculating a first extreme difference a between the weight average of the edge zones S3 and the weight average of the center zone S2 based on the weight average of the edge zones S3 and the weight average of the center zone S2, comparing the first extreme difference a with a first specified extreme difference A, and determining, based on a>A, that coating on a surface of the membrane has warped edges and/or thick edges; where the first specified extreme difference A is set to 5.5 mg; and
calculating a weight extreme difference b of the primary zone S 1, comparing the weight extreme difference b with a second specified extreme difference B, and determining, based on b>B, that the coating on the surface of the membrane has dark traces and/or scratches; where the second specified extreme difference B is set to 8 mg.

In conclusion, it should be noted that the above embodiments are merely intended for describing the technical solutions of this application but not for limiting this application. Although this application is described in detail with reference to the foregoing embodiments, persons of ordinary skill in the art should understand that they may still make modifications to the technical solutions described in the foregoing embodiments or make equivalent replacements to some or all technical features thereof without departing from the scope of the technical solutions of the embodiments of this application. They should all be covered in the scope of the claims and specification of this application. In particular, as long as there is no structural conflict, the various technical features mentioned in the implementations can be combined in any manners. This application is not limited to the specific implementations disclosed in this specification, but includes all technical solutions falling within the scope of the claims.

## Claims

1. A coating quality detection method, used for detecting quality of a coated membrane and **characterized in that** the method comprises the following steps:
detecting a coating region on the membrane to obtain millimeter wave data;
obtaining edge zero lines on two sides of the coating region, and setting a primary zone of the coating region based on the edge zero lines on the two sides, wherein the primary zone comprises a center zone located at the center thereof and edge zones located at the edges thereof;
calculating a weight average of the edge zones and a weight average of the center zone;
calculating a first extreme difference a between the weight average of the edge zones and the weight average of the center zone based on the weight average of the edge zones and the weight average of the center zone, comparing the first extreme difference a with a first specified extreme difference A, and determining, based on a>A, that coating on a surface of the membrane is abnormal; and/or
calculating a weight extreme difference b of the primary zone, comparing the weight extreme difference b with a second specified extreme difference B, and determining, based on b>B, that the coating on the surface of the membrane is abnormal.

2. The coating quality detection method according to claim 1, wherein the step of obtaining edge zero lines on two sides of the coating region comprises:
setting any point as an edge zero point of the coating region based on transition of a weight value of the point on the coating region to a specified weight value; and
determining an edge zero line based on a plurality of edge zero points on the same side of the coating region, wherein the plurality of edge zero points on the same side are jointly set on the edge zero line on the same side.

3. The coating quality detection method according to claim 2, wherein the specified weight value is set to 250 mg.

4. The coating quality detection method according to claim 3, wherein a line midway between the edge zero lines on the two sides is set as a center line, and a region formed by translating the center line towards the edge zero lines on the two sides by a length of L1 is the center zone, wherein 0<L1≤5 mm.

5. The coating quality detection method according to claim 4, wherein regions formed by separately translating boundary lines on two sides of the primary zone towards the center of the membrane by a length of L2 are the edge zones, wherein 0<L2≤10 mm.

6. The coating quality detection method according to claim 5, wherein the edge zero lines on the two sides are separately translated towards the center of the membrane by a length of L3 to form thinning zones, wherein the thinning zones are arranged adjacent to the primary zone, and 0≤L3≤10 mm.

7. The coating quality detection method according to claim 6, wherein a remaining region after the thinning zones between the edge zero lines on the two sides are excluded is set as the primary zone.

8. The coating quality detection method according to any one of claims 1 to 7, wherein the first specified extreme difference A is set to 5.5 mg, and/or the second specified extreme difference B is set to 8 mg.

9. The coating quality detection method according to any one of claims 1 to 7, wherein the determining, based on a>A, that coating on a surface of the membrane is abnormal comprises determining that the coating on the surface of the membrane has warped edges and/or thick edges.

10. The coating quality detection method according to any one of claims 1 to 7, wherein the determining, based on b>B, that the coating on the surface of the membrane is abnormal comprises determining that the coating on the surface of the membrane has dark traces and/or scratches.

11. The coating quality detection method according to any one of claims 1 to 7, wherein the detection method further comprises the following step:
performing difference operation on the millimeter wave data of the coating region.

12. The coating quality detection method according to any one of claims 1 to 7, wherein after the determining that coating on a surface of the membrane is abnormal, the method further comprises the following step:
issuing an alarm prompt, and/or marking a region on the surface of the membrane as abnormal coating.

13. A coating quality detection apparatus, configured to perform the coating quality detection method according to any one of claims 1 to 12, wherein the detection apparatus comprises:
a detection module, configured to detect a coating region on a membrane to obtain millimeter wave data;
an obtaining module, configured to: obtain edge zero lines on two sides of the coating region, and set a primary zone of the coating region based on the edge zero lines on the two sides, wherein the primary zone comprises a center zone located at the center thereof and edge zones located at the edges thereof;
a calculation module, configured to: calculate a weight average of the edge zones and a weight average of the center zone, and calculate a first extreme difference a between the weight average of the edge zones and the weight average of the center zone based on the weight average of the edge zones and the weight average of the center zone, and configured to calculate a weight extreme difference b of the primary zone; and
a determining module, configured to: compare the first extreme difference a with a first specified extreme difference A, and determine, based on a>A, that coating on a surface of the membrane is abnormal, and/or configured to: compare the weight extreme difference b with a second specified extreme difference B, and determine, based on b>B, that the coating on the surface of the membrane is abnormal.

14. The coating quality detection apparatus according to claim 13, wherein the detection module comprises an X-ray surface density measuring instrument.

15. A coating quality detection system, wherein the detection system comprises a processor and a memory storing computer program instructions; and
wherein when the computer program instructions are executed by the processor, the coating quality detection method according to any one of claims 1 to 12 is implemented.

16. A storage medium, wherein the storage medium stores computer program instructions, and when the computer program instructions are executed by a processor, the coating quality detection method according to any one of claims 1 to 12 is implemented.
